# EUROPEAN PATENT APPLICATION

(11) **EP 4 768 561 A1**
(43) Date of publication of application: **01.07.2026**
(21) Application number: 24223296.5
(22) Date of filing: 26.12.2024
(51) Int. Cl.: C11D 3/33, C11D 3/36, C11D 11/02, A61Q 5/00, A61Q 11/00, A61Q 19/10

(54) **SYNERGISTIC COMPOSITIONS OF HEDP AND MGDA/GLDA FOR IMPROVED SEQUESTERING AGENT POWDER PRODUCTION**

(71) Applicant: MKS Devo Kimya Sanayi Ticaret Anonim Sirketi, Istanbul (TR)
(72) Inventor: SENGUN, Mehmet Korgun, Istanbul (TR)
(74) Representative: Mutlu, Aydin

(57) **Abstract**

The present invention relates an improved sequesterant composition in powder or granule form comprising HEDP and an amino acid based sequesterant selected from the group of MGDA and GLDA. In other aspects, the invention relates to a method for the preparation of said compositions as well as their use in cleaning products.

## Description

### Technical Field of the Invention

The present invention relates to the development of sequestering agents for industrial and domestic use. Specifically, the invention concerns powdered compositions of hydroxyethylidene diphosphonic acid (HEDP) and amino acid-based chelating agents, including methylglycine diacetic acid (MGDA) and glutamic acid diacetic acid (GLDA), optimized for superior performance and reduced environmental impact.

### Background of the Invention

Sequestering agents are widely used to remove soluble or slightly soluble inorganic ions, metals, and their salts from water sources. These agents are commonly applied in industrial settings -such as boilers, reverse osmosis systems, cooling towers, and heat exchangers - and in household products, including detergents, washing agents, heavy-duty cleaners, bathroom and kitchen cleaners, shampoos, toothpaste, and bleaching solutions. Their primary purpose is to prevent the formation of undesirable deposits or scales in water systems caused by metal ions such as calcium (Ca²⁺), magnesium (Mg²⁺), and iron (Fe²⁺/Fe³⁺). Sequestering agents can have anionic, nonionic, or mixed anionic/nonionic structures and may exist as polymers, oligomers, macromolecules, or single molecules. Most commonly, they function by having anionic groups that bind to cationic or non-ionized inorganic substances and dispersible particles via electrolytic forces. This interaction either dissolves these materials or renders them incapable of forming scales in water systems such as pipes, pipelines, and heat exchangers.

The sources of sequestering agents can vary and may be natural, semi-synthetic, or fully synthetic, such as citric acid, polyaspartic acid, and polyacrylic acid. These agents are available in solid or liquid form, and their usage depends on the application. For instance, in a reverse osmosis system, a liquid sequestering agent is continuously dosed into the water supply, whereas powdered forms are required for products like washing machine detergents to ensure compatibility with the formulation.

Detergents and cleaners are produced for both industrial and retail purposes. Over the years, numerous types of detergents and cleaners have been developed, including laundry detergents, heavy-duty detergents, hard surface cleaners, toilet bowl cleaners, bathroom cleaners, kitchen cleaners, personal care products, glass cleaners, and bleaches (chlorine and non-chlorine). Some of these products are ready-to-use (e.g., glass cleaner, toilet bowl cleaner, bathroom cleaner, kitchen cleaner, personal care products), while others must be diluted in water. However, water used in these formulations often contains various soluble elements, including Ca, Mg, Cd, Cr, Cu, Fe, Pb, Co, Mn, Hg, Ni, Zn, Al, As, Si, and S, depending on the water source.

The formulations of these products typically include surfactants, fillers, builders, sequestrants, boosters, solvents, hydrotropes, fragrances, enzymes, bleachers, bleach activators, pH adjusters, dyes, preservatives, and other additives. Formulations must remain stable throughout their shelf life. For example, in non-chlorine bleach formulations, hydrogen peroxide serves as an oxidative agent but can destabilize in the presence of trace metals such as iron. This instability can lead to decomposition, releasing oxygen gas, which causes bottles swelling during storage. The addition of appropriate sequestering agents can chelate these free metals, preventing such adverse reactions.

Chelating agents function by coordinating with metal ions through donor atoms such as oxygen, nitrogen, sulfur, or phosphorus. The specific arrangement and properties of these atoms give each chelating molecule unique capabilities. Common chelating agents include GLDA, MGDA, EDTA, ATMP, HEDP, PBTC, DTPMP, CA, SC, PAA, SHMP, SPA, and PMA.

Over time, the development of chelating agents has been driven by environmental concerns, cost considerations, greener production methods, reduced carbon footprints, and ecological safety. For instance, environmental issues associated with phosphates led to the development of phosphonates. Subsequently, phosphonates have come under scrutiny for their environmental impact, leading to the promotion of more natural and biodegradable agents like MGDA (methylglycinediacetic acid) and GLDA (glutamic acid N,N-diacetic acid).

GLDA and MGDA are modern, biodegradable chelating agents that are not classified as hazardous. They effectively bind calcium and magnesium ions, which are responsible for many types of stains. These agents can remove hard water carbonate deposits by converting them into water-soluble chelates, thereby enhancing the effectiveness of anionic surfactants. GLDA, for example, is produced from carbohydrate sources such as sugar, molasses, corn, or rice using biochemical processes that yield a highly biodegradable L-isomer.

Although GLDA and MGDA have ecological advantages, they generally demonstrate lower chelation performance compared to phosphonates like HEDP. Additionally, their production is more costly, and the limited availability of raw materials, such as amino acids, poses supply chain challenges, particularly due to dependence on specific sources.

Sequestering agents are indispensable in formulations containing anionic surfactants, such as linear alkyl benzene sulfonates, fatty acid salts, and alkyl sulfates. These surfactants are vulnerable to water hardness, where calcium and magnesium ions replace sodium ions, reducing their surface-active properties. Consequently, sequestering agents are critical in products like laundry detergents.

The demand for powdered sequestering agents has grown significantly, particularly for powdered detergents and dishwasher detergents. Producing powdered agents involves drying techniques like spray drying, rotary drying, and fluidized bed drying. These processes require substantial energy, and the efficiency varies based on factors such as molecular structure and bonding forces. For instance, producing 50% MGDA powder at 250-350°C yields 200 kg of product per batch, whereas producing the same quantity of HEDP powder at 180-250°C yields 400 kg.

In applications such as dishwashing detergent tablets, phosphorus content is limited. Tablets typically contain 20-80 wt % chelating agents, with higher bulk densities (400-1000 kg/m³) preferred to reduce tablet volume. Two common tablet types include those wrapped in water-soluble films (e.g. PVA) and those compressed into a solid form. Both aim to optimize space while maintaining efficacy.

EP3484988 describes MGDA and GLDA formulations optimized for cleaning and sequestering applications. The patent emphasizes the ecological benefits of MGDA and GLDA, including biodegradability and compatibility with environmental concerns, and adresses yellowing problems of cleaning compositions including oxygen bleach. This document, however, does not propose blending MGDA with phosphonates like HEDP to address MGDA's weaker chelation performance and higher production costs. The patent also overlooks energy efficiency in powder production.

WO2012168739 describes builder granules of MGDA produced under non-agglomerating conditions using spray drying. The focus is on achieving uniform particle sizes and avoiding aggregation. The scope of this document is limited to MGDA complexing agent blends, with no exploration of phosphonate interactions or energy optimization in drying processes.

WO2015036324 highlights various salts and isomers of MGDA used in cleaning formulations. This document emphasizes the role of specific isomers in enhancing performance. Nonetheless, it does not address bulk density improvements or synergistic blending for better chelation and energy efficiency.

US20170058239 explores granulation processes for MGDA and GLDA at high solid contents to produce powders and granules. It discusses maintaining high purity and low moisture content The document does not optimize bulk density or address synergy with HEDP to reduce drying temperatures.

WO2015121170 discloses blends of MGDA and GLDA with homo- or copolymers of (meth)acrylic acid, emphasizing compatibility with polymeric systems. The document does not extend to synergistic blends involving HEDP.

WO2009103822 focuses on producing solid MGDA granules using heat-assisted processes. It addresses issues with particle size and agglomeration and aims at provision of free flowing granules. The document does not address issues relating to bulk density or cost reductions through synergistic blending.

WO2020094480 explores MGDA and GLDA blends for granulation processes, including specific ratios. It emphasizes also achieving homogeneity and stability. The document does not consider combinations with HEDP or the resulting benefits in bulk density and operation costs.

There is an unmet need in state of the art for an optimized composition that leverages the strengths of HEDP, MGDA and GLDA while mitigating their individual drawbacks. These and other drawbacks of the conventional sequestering agents are eliminated with novel compositions, processes and specific uses as defined in the appended claims.

### Brief Description of the Invention

The present invention relates to an improved composition in powder or granule form comprising HEDP (1-hydroxyethylidene-1,1-diphosphonic acid) and an amino acid based sequesterant selected from the group of MGDA (methylglycinediacetic acid) and GLDA (glutamic acid N,N-diacetic acid). Said amino acid based sequesterant is preferably MGDA.

In an other aspect, the invention provides a method for the preparation of a sequesterant composition comprising blending of HEDP with an amino acid based sequesterant selected from GLDA and MGDA, and drying the same to obtain a composition in powder form. This method may further comprise a granulation step. The drying is preferably carried out in a spray drier.

In a further aspect, the invention provides a use of the aforementioned sequesterant composition in a cleaning composition. The cleaning composition is preferably selected from the group consisting of dishwasher detergents, laundry detergents, hard surface cleaners, toilet bowl cleaners, bathroom cleaners, kitchen cleaners, personal care products, shampoos, toothpaste, glass cleaners, and bleaches. Most preferably, the cleaning composition is dishwasher detergent

### Detailed Description of the Invention

Many traditional sequestering agents, such as phosphonates, contribute significantly to environmental phosphorus loads, resulting in issues like eutrophication in aquatic ecosystems. Regulatory restrictions on phosphorus content in detergents and other cleaning agents necessitate the development of environmentally friendly alternatives. On the other hand, high production costs and energy-intensive manufacturing processes are common challenges for widely used sequestering agents like MGDA and GLDA. Dependence on limited sources for raw materials such as amino acids creates supply chain vulnerabilities, particularly for eco-friendly agents like MGDA and GLDA. While HEDP demonstrates superior chelation strength, its biodegradability lags compared to MGDA and GLDA. Conversely, MGDA and GLDA, while more biodegradable, exhibit lower calcium-binding efficiency, which limits their practical applications in high-performance scenarios.

Recent years have witnessed a shift toward greener and more sustainable sequestering agents. Regulatory frameworks in Europe and beyond have imposed strict limits on phosphorus content in household and industrial products. Additionally, consumer preference for biodegradable and non-toxic products has driven innovation toward amino acid-based agents like MGDA and GLDA.

However, achieving competitive performance with these greener alternatives requires overcoming several hurdles, including:
- Low bulk density, which affects transportation and formulation efficiency.
- High energy consumption during spray drying processes, increasing production costs.
- Relatively weak chelation performance compared to conventional phosphonates.

The invention described herein bridges these gaps by introducing a novel composition of HEDP with MGDA and/or GLDA. This innovative approach leverages the strengths of each component to achieve:
- Reduced production energy requirements through lower drying temperatures.
- Improved bulk density, facilitating efficient storage and transportation.
- Enhanced calcium-binding capacity, rivaling conventional agents while maintaining superior biodegradability.

By addressing these challenges comprehensively, the proposed invention not only meets but exceeds the expectations of modern industrial and household applications.

In an aspect, the present invention provides a sequestering agent composition in powder or granule form comprising HEDP (1-hydroxyethylidene-1,1-diphosphonic acid) as a phosphonate together with an amino acid based sequesterant selected from the group of MGDA (methylglycinediacetic acid) and GLDA (glutamic acid N,N-diacetic acid). Most preferably, the amino acid based sequesterant is composed of MGDA.

In the present context, HEDP, MGDA and GLDA, can either be their free organic form or alkali metal salts thereof. HEDP can be used in the form disodium salt, while MGDA and GLDA can be used in the form of trisodium salt and tetrasodium salt, respectively. Likewise, MGDA and/or GLDA can be provided as a mixture of L- and D- enantiomers thereof. More preferably, MGDA and/or GLDA is provided in a form of enriched by L-isomer or as a mixture of recemate. More preferably L-isomer of the MGDA and/or GLDA constitute about 50-80% by weight of the overall enantiomeric mixture.

Etidronic acid, also known as HEDP (1-hydroxyethylidene-1,1-diphosphonic acid), is an organophosphonate compound commonly used as a chelating agent and water treatment chemical. It contains a central ethylidene group attached to two phosphonic acid groups. Hydroxyl group (-OH) and phosphonic acid groups (-PO₃H₂) contribute to its chelating and sequestration ability. It is highly soluble in water, stable under normal conditions but decomposes upon heating at high temperatures. HEDP binds metal ions through the oxygen atoms of the phosphonic acid groups and the hydroxyl group. The resulting chelates are water-soluble and stable, preventing the precipitation of metal ions. HEDP is favored for its cost-effectiveness and good balance of chelation and stability. However, it is not sufficiently biodegradable and is therefore less eco-friendly.

MGDA is a biodegradable aminopolycarboxylate chelating agent. Its structure features an amine group (-NH) bonded to a carboxymethyl group (-CH₂COOH), which provides the functional groups necessary for chelation. MGDA is known for its high biodegradability compared to other chelating agents like EDTA or DTPA. This makes MGDA an environmentally friendly choice, especially in industrial and domestic cleaning applications. MGDA exhibits good chemical stability in a wide range of pH values, making it suitable for both acidic and alkaline conditions. It is thermally stable under standard operating conditions. MGDA is highly soluble in water, which enhances its performance in aqueous systems. It has low toxicity to humans and aquatic organisms, supporting its use in environmentally sensitive applications. MGDA is compatible with various cleaning formulations, including detergents, metal-cleaning products, and personal care items. However, it has shortcomings in terms of chelation performance as compared to HEDP.

GLDA is a biodegradable, eco-friendly chelating agent derived from natural amino acids. It is increasingly used as a substitute for conventional chelating agents like EDTA, NTA, and DTPA due to its superior environmental profile and excellent chelation performance. GLDA is a derivative of the amino acid glutamic acid. It contains multiple carboxylic acid groups and amine functionalities, enabling effective coordination with metal ions. Its structure provides high solubility in water and compatibility with a range of pH conditions. GLDA is readily biodegradable, meeting stringent environmental standards. It breaks down into non-toxic substances, minimizing its environmental footprint It is derived from renewable raw materials (e.g. plant-based sources) and is less harmful to aquatic ecosystems compared to traditional chelating agents. GLDA exhibits excellent stability under varying temperature and chemical conditions, maintaining its chelating properties in harsh environments. It has low acute toxicity to humans and aquatic organisms, making it safer for industrial and consumer applications. GLDA effectively binds to a wide range of metal ions, including calcium, magnesium, iron, copper, and heavy metals. It forms stable complexes, even in hard water and high pH environments. GLDA performs well across a wide pH range (2-13), making it versatile for various industrial and cleaning applications. GLDA demonstrates particularly strong chelation of iron ions (Fe³⁺ and Fe²⁺), making it ideal for water treatment, detergents, and fertilizers. It is compatible with various detergents, surfactants, and cleaning agents, enhancing their performance by sequestering metal ions. However, it has shortcomings in terms of chelation performance as compared to HEDP.

In preferred embodiments, the ratio of HEDP:(GLDA and/or MGDA) ranges, for instance, from 95:5 to 50:50, by weight In more preferred embodiments, the mixture is provided with a HEDP:(GLDA and/or MGDA) weight ratio between 90:10 to 55:45. Particular effects were observed with a composition exhibiting a HEDP:MGDA weight ratio ranging from 90:10 to 60:40.

The composition according to the present invention has a bulk density ranging from 750 kg/m³ to 950 kg/m³, more preferably from 800 kg/m³ to 900 kg/m³.

The compositions according to the present invention can be provided in powder or granulated forms. In another aspect, the present invention pertains to a method for preparing a sequestering agent composition comprising blending of HEDP with an amino acid based sequesterant selected from GLDA and MGDA, and drying the same to obtain a composition in powder form.

In another aspect, the present invention provides specific uses of the aforementioned composition as a sequestering agent in a cleaning product selected from the group consisting of dishwasher detergents, laundry detergents, hard surface cleaners, toilet bowl cleaners, bathroom cleaners, kitchen cleaners, personal care products, shampoos, toothpaste, glass cleaners, and bleaches. The drying step is preferably performed at a temperature ranging from 250°C to 300°C.

The compositions according to the present invention provide several advantages in terms of the process temperature, alkali binding capacity and bulk density as demonstrated in the following examples.

### Example 1 - Preparation of the Standard Powdered Formulation

A slurry containing 50% solids is prepared by blending HEDP and MGDA in a weight ratio of 72:28. The slurry is dried using a spray dryer to give a powdered composition. Control samples were prepared by using HEDP and MGDA without HEDP. Total amounts of sequestering agents in each composition were the same.

### Example 2 - Process Temperature

Slurries having 50 wt % solid material were prepared according to Example 1 and fed to a sprey dryer whereby the evaporation temperature necessary for obtaining completely dried material in poweder form (moisture content < 1 wt%) within a predetermined fixed time interval was determined for each sample.

**Table1. Process temperature effect**

| **Material** | **Solid matter (wt** %) | **Evaporation temperature (°C)** |
|---|---|---|
| MGDA | 50 | 350 |
| HEDP | 50 | 250 |
| HEDP + MGDA | 50 | 258 |

The results were promising as the evaporation temperature was not substantially affected by the MGDA content in the HEDP + MGDA mixture. Therefore, contrary to the expectations, the usage of MGDA doesn't cause a substantial effect in power consumption in the spray drier.

### Example 3 - Ca++ Binding Capacity

Slurries having 50 wt % solid material were prepared according to Example 1 and tested in terms of the Ca++ binding capacity.

**Table 2. Ca++ binding capacity**

| **Material** | **Solid matter (wt** %) | **Ca++ binding** |
|---|---|---|
| MGDA | 50 | 380 |
| HEDP | 50 | 680 |
| HEDP + MGDA | 50 | 638 |

Table 2 shows that Ca++ binding capacity of the mixture is almost in average of the individual sequesterants MGDA and HEDP. This means the composition perfectly matches the performance point Complexing agents for use as sequestrants commonly characterized by their calcium binding capacity, which is a measure for the amount of calcium bound by a given amount of complexing agent at a given pH and temperature. Calcium binding capacity was monitorized by the Hampshire method. MGDA and HEDP application performances were determined at pH 11 and 20 °C.

### Example 3 - Bulk Density

Slurries having 50 wt % solid material were prepared according to Example 1 and spray dried as defined in Example 2 to obtain the composition in powder form. The powders were tested with respect to bulk densities.

**Table 3. Bulk density**

| **Material** | **Bulk density (kg/m³)** |
|---|---|
| MGDA | 700 |
| HEDP | 500 |
| HEDP + MGDA | 850 |

Table 3 shows a clear synergism of HEDP + MGDA as compared to individual sequesterants. Bulk density was unexpectedly increased as a result of co-formulation and drying of HEDP and MGDA. Without wishing to be bound by a theory, it is thought that MGDA and HEDP perfectly match in particle level to fill the voids and gaps, and thereby increasing the overall density of the blend. Bulk density was measured according to ASTM D6683 Standard Test Method for Measuring Bulk Density Values of Powders and Other Bulk Solids as Function of Compressive Stress.

Further advantages of the composition involve easy shaping of the powders with almost all types of dryers. Biodegradability dramatically increases, and total phosphorous content is reduced below the upper limits while achieving high level calcium binding capacity with very high bulk density. As mentioned above, the composition according to the invention offers also lower consumption of energy, lower carbon footprint, lower costs, and as a result, higher environmental and commercial benefits.

## Claims

1. A composition in powder or granule form comprising HEDP (1-hydroxyethylidene-1,1-diphosphonic acid) and an amino acid based sequesterant selected from the group of MGDA (methylglycinediacetic acid) and GLDA (glutamic acid N,N-diacetic acid).

2. The composition according to claim 1 wherein the ratio of HEDP:(GLDA and/or MGDA) ranges from 95:5 to 50:50, by weight

3. The composition according to claim 1 wherein the ratio of HEDP:(GLDA and/or MGDA) ranges from 90:10 to 55:45, by weight

4. The composition according to claim 1 wherein the ratio of HEDP:(GLDA and/or MGDA) ranges from 50:50 to 60:40, by weight

5. The composition according to claim 1 wherein bulk density of the composition ranges from 750 kg/m³ to 950 kg/m³.

6. The composition according to claim 5 wherein bulk density of the composition ranges from 800 kg/m³ to 900 kg/m³.

7. The composition according to claim 1 wherein amino acid based sequesterant is MGDA.

8. A method for the preparation of a sequesterant composition comprising blending of HEDP with an amino acid based sequesterant selected from GLDA and MGDA, and drying the same to obtain a composition in powder form.

9. The method according to claim 8 further comprising granulation of the powders obtained according to claim 8.

10. The method according to claim 8 wherein drying is carried out in a spray drier.

11. The method according to claim 8 wherein drying is performed at a temperature ranging from 250°C to 300°C.

12. The method according to claim 8 wherein amino acid based sequesterant is MGDA.

13. Use of the composition according to any of the claims 1 to 7 as a sequesterant in a cleaning composition.

14. The use according to claim 13 wherein the cleaning composition is selected from the group consisting of dishwasher detergents, laundry detergents, hard surface cleaners, toilet bowl cleaners, bathroom cleaners, kitchen cleaners, personal care products, shampoos, toothpaste, glass cleaners, and bleaches.

15. The use according to claim 14 wherein the cleaning composition is dishwasher detergent
